# EUROPEAN PATENT APPLICATION

(11) **EP 4 012 974 A1**
(43) Date of publication of application: **15.06.2022**
(21) Application number: 21213717.8
(22) Date of filing: 10.12.2021
(51) Int. Cl.: H04L 9/32, G06K 19/06, G16H 10/65

(54) **CRYPTOGRAPHICALLY VERIFIABLE, PORTABLE CERTIFICATE**

(30) Priority: 10.12.2020 US 202063124036 P
(71) Applicant: Guardtime SA, 1006 Lausanne (CH)
(72) Inventor: Day, Garrett, Arlington, VA, 22201 (US); Raymond, Gregory, Tampa, FL 33611 (US)
(74) Representative: Grünecker Patent- und Rechtsanwälte PartG mbB

(57) **Abstract**

A vaccination certificate (300) is provided with at least one optically readable marking (600) that encodes a digital signature of the identity of a patient (320), and well as data relating to a vaccine and its administration to the patient. The digital signature enables verification of purportedly correct information without needing to know the personal identifying information of the patient, and without querying an external database. A salt value may also be added to the patient identity information to increase entropy in the digital signature.

## Description

### BACKGROUND

The Covid-19 pandemic has revealed an urgent need for a global and cross-sector response to mitigate the spread of communicable diseases, which are capable of severe global impact. Vaccination against the virus is expected to be one of the core strategic approaches to this end, as it reduces both the individual risk of carrying and spreading the infection as well as protecting public health by reducing the probability of disease spread.

Implementing an effective global vaccination program against SARS-CoV-2 for 7.7 billion people in every corner of the world within is perhaps one of the most complex endeavors ever undertaken. It may be the largest product launch in human history, and poses huge risks for public officials as well as for the companies involved in this undertaking.

Roll-out must balance equitable access in the initial shortage phase and overcome scepticism expressed by large part of the population later on. A robust public health monitoring system is required to steer this effectively. It is important to keep track of vaccinations at the individual level (e.g. multiple doses, side-effects etc.) as well as at the population level (priority management, uptake and allocation fairness etc.). Quality monitoring (pharmacovigilance) is more important than ever due to record speed of development, the number of totally new technologies used for Covid19 vaccines, and the sheer number of competing vaccines that are being deployed simultaneously.

On the other hand, manufacturing and distributing the vaccines on this scale around the world requires logistical planning and cooperation of multiple organizations within and across countries with clockwork precision. Government authorities and private companies need to work closely together while maintaining integrity of their respective roles. Good and reliable information becomes vital for maintaining public trust under such circumstances.

Equally important is the need to establish a system that will allow reliable verification of being immunized. As the need to go back to normal activities and travel is overwhelming there will be a huge incentive for bad actors in public and private organizations, as well across the medical supply chain, to produce fraudulent attestations and incorrect procedures to counterfeit and divert vaccines.

Reopening the economy locally as well as globally requires any vaccination certificate solution to provide assurance that the individual has been truly and effectively vaccinated. This requires a reliable link between the individual, the specific (functional) vaccine and a trustworthy healthcare institution where the vaccination was given.

Many different solutions for vaccination attestation are of course being proposed around the world, and many are already in widespread use. Many of these build on some kind of certificate that bears a code that is assigned to a patient after vaccination. One problem with these solutions is that it is often easy to simply copy the certificate, code and all, and pass it off as belonging to another. In some jurisdictions such as the USA, the official vaccination certificate itself is particularly easy to copy, or to counterfeit, or digitally alter (for uploading) since they do not even bear any form of encrypted information.

Some attempt to eliminate this weakness by having a verifier refer to an external database to check the identity of the patient. One weakness of this is precisely the need to have such a database, which is difficult to establish and maintain, or may encounter distrust and resistance among the population.

### BRIEF DESCRIPTION OF THE DRAWINGS

Figure 1 is a flowchart that illustrates how the medical procedure of vaccine administration may be linked with the issuance of a trusted certificate about the fact.
Figure 2 illustrates information capture and entry in the context of vaccination certification.
Figure 3 illustrates the procedure of provably recording information.
Figure 4 depicts an example of a vaccination certificate that reveals the name of the patient.
Figure 5 illustrates how an embodiment of the invention may be used to implement a supply chain information management platform.
Figure 6 an example of a cryptologically secured and verifiable vaccination certificate that does not reveal the name of the patient in human-readable form.

### DESCRIPTION

Embodiments of this invention provide a solution that enables monitoring of the supply-chain of a product in real time, connected by certificates whose contents are verifiable, as well as the identity of the holder. By way of example, embodiments are described for a particularly relevant and timely use case, namely, vaccination certificates. For the sake of brevity, embodiments relating to this use case are referred to as "VaccineGuard".

VaccineGuard may be applied worldwide with uniform security, both on paper and with modern digital platforms, regardless of a country's level of digital development. The proposed solution can be integrated with existing, proprietary IT systems to ensure fast implementation of the solution. Although VaccineGuard may be implemented on a limited, local scale, it is possible to link multiple systems that are all integral to vaccination delivery into a common reliable information and communication space - locally and worldwide.

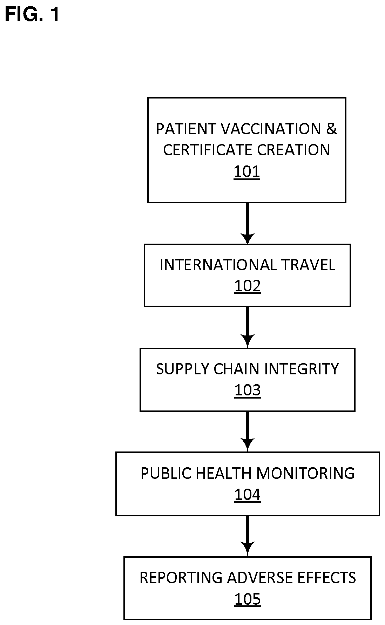

Figure 1 illustrates how the medical procedure of vaccine administration may be linked with the issuance of a trusted certificate about the fact. The certificate may be used worldwide to verify vaccination status, for example, if this is required to cross an international border or access a high-risk facility. Figure 1 shows the steps of one example of the procedure made possible by VaccineGuard:

### 101: PATIENT VACCINATION & CERTIFICATE CREATION

A healthcare worker administers the vaccination to Patient and provides Patient with a vaccination certificate, which can be on any suitable medium, such as paper with a scannable QR code or as an application on patient's smart phone. In this step, at least some of the patient's PII must be shared in order to create the certificate.

### 102: INTERNATIONAL TRAVEL

Patient, in the USA, wishes to meet friends in the UK. Patient presents his vaccination certificate to a Border Control officer to prove that he has received the appropriate vaccination. The officer scans the smart vaccination certificate according to this invention to validate. Note that there is NO need to share PII in this step.

### 103: SUPPLY CHAIN INTEGRITY

The vaccination certificate may also be provided with information that overlaps with supply chain data. Again, PII sharing is NOT needed

### 104: PUBLIC HEALTH MONITORING

The healthcare worker administers the vaccine to the Patient and logs it onto the user's vaccination certificate. The information can also be extracted and de-identified and automatically submitted for monitoring purposes by the Health Authority. Again, PII sharing is NOT needed.

### 105: REPORTING ADVERSE EFFECTS

Patient feels ill after receiving the vaccination, goes to hospital and provides the doctor with Patient's vaccination certificate. The doctor then populates any specified reporting form with data scanned from Patient's vaccination certificate. For this step, PII sharing is needed.

Vaccine distribution (supply-chain) requires up-to-date information to avoid shortages or to discover counterfeits. Further, public health reporting is vital to coordinate effective and equitable vaccination campaign roll-out. Eventually, all the previous information needs to be available for investigation in case unwanted adverse events (such as patient reactions) are discovered. VaccineGuard may be used to connect multiple systems that are all integral to vaccination delivery. The solution allows different interlinked stakeholders to execute their tasks and for companies and governments responsible for managing the rollout to obtain real-time insights on the plans' performance, based on reliable information. At the same time, VaccineGuard maintains the highest level of security and privacy protections for sensitive data while supporting each respective role - individuals, public authorities or manufacturers - in their tasks with single version of reliable information.

For users (referred to here as "patients", since their initial interaction with the system will typically be as patients being vaccinated), VaccineGuard provides a globally valid, digitally verifiable vaccination certificate to enable safe travel and confidence in the authenticity of the vaccine used, as well as rapid feedback during a vaccine's quality-monitoring / pharmacovigilance period (for example, notification when new information about the vaccine's effectiveness becomes available, etc.).

For Public Health Authorities that are responsible for a vaccination campaign, VaccineGuard enables an operational overview of the implementation of the vaccination campaign within their jurisdiction, but also internationally for effective collaboration.

Similarly, vaccine manufacturers may receive anonymous real-time insight about supply-chain effectiveness (where are vaccines admitted, level of available stocks) as well as early warning on diversion (when vaccines intended for one country surface in another place) or counterfeit (when vaccinations are performed with non-authentic vaccines).

For all the involved parties, VaccineGuard enables effective quality monitoring and mitigation support by facilitating high-accuracy pharmacovigilance data collection, analysis, and rapid distribution to a relevant entity. Public health authorities may receive early warning that can be communicated to manufacturers for rapid mitigation measures; and citizens may get timely notification if new information becomes known about their vaccination appointments. This may be achieved by anonymous reporting that is securely linked to unique vaccination records and supply chain process artefacts using the same cryptographic method that protects the reliability of certificates as well as the privacy of individuals.

The certificate ("VaccineGuard Certificates") created by VaccineGuard protects data accuracy at the moment of creation:
- The provider of vaccination services as well as the issuer of the vaccination certificate is preferably an authorized healthcare or health data service provider. Such institutional authorization is typically provided by a government or other internationally recognized entity. VaccineGuard certificates preferably originate only from an Issuer that belongs to a trusted list, which can be digitally verified; this provides a trust framework.
- The vaccine used for immunization and creation of a vaccination certificate is preferably verified against an authentic vaccine data repository. This is done using the "digital twin" of each vaccine created, together with, for example, the physical vaccine vial, which are defined via unique serial numbers (see below). VaccineGuard certificates are issued with the reference to a unique authentic vaccine, which can be digitally verified.
- The vaccine recipient (the "user") is authenticated by the healthcare provider using existing photo-ID that is cryptographically linked to the vaccination certificate. Thus, a VaccineGuard certificate can only be issued with a reference to a unique individual, which can be digitally verified. If needed, also verification of a person against a prioritization and eligibility database can be facilitated.

To summarize, each VaccineGuard vaccination certificate creates a reliable link between a specific individual, an authentic vaccine dose and a trusted healthcare institution. No vaccine can be marked as used unless an authenticated unique individual is attached to it and no individual can be marked for being vaccinated unless an authentic unique vaccine is attached to it.

In one embodiment, a "whitelist" of trusted healthcare providers is established. Trusted healthcare providers are preferably authorized to perform vaccinations by, for example, some governmental body or recognized organization. VaccineGuard preferably extends access to its services for the digital certificate attestation only to those on the whitelist, or who are otherwise authorized. A dedicated digital "trust framework" may be used to facilitate this component.

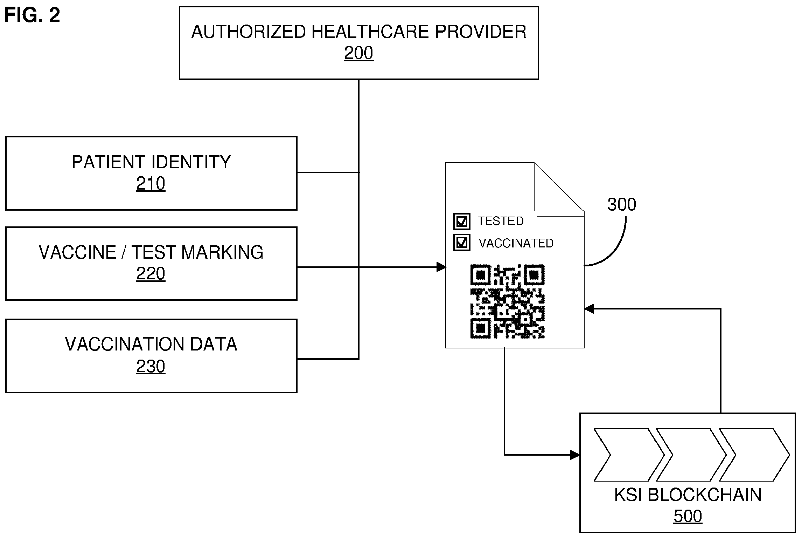

Second, the data describing vaccination of an individual - personal details, administered vaccine, relevant contextual information - is then captured by the trusted healthcare provider, who guarantees that the data entry is accurate. Figure 2 illustrates this information capture and entry in the context of vaccination certification. Information that may be included in a certificate will typically include:
a. The identity and, in most cases, some form of national authorization identifier of an authorized healthcare provider 200
b. Patient identity information 210, which may established using any conventional means, such as by a national ID card, a passport, or some other approved document
c. A vaccine and/or testing marking 220, which may follow any known protocol such as GS1 or HL7
d. Vaccination data 230, such as according to the IHR or FHIR protocols

This information is then preferably submitted to a system to receive a corresponding cryptographic proof. In the illustrated and preferred embodiment, this system is the known KSI blockchain 500, which is described in more detail below. VaccineGuard thus provides an immutability guarantee to the vaccination certificate, in the preferred case by obtaining a KSI signature for the included information, which is such that if even a single data point - indeed, a single digital bit -- in any of the certificates fails verification it is possible to trace back the information on the issuer or the vaccine that is causing disqualified verification. Note that, by using the KSI system, this will be possible even without keeping a centralized registry of all vaccinations and without processing personally identifiable information (PII). Such immutability guarantee assures reliability of both the data and process that was used to generate it for any later use of this information by vaccination certificates or supply chain monitoring.

Because the components of the vaccination attestation are processed independently - and can be separated from the attestation document itself - they can be used for legitimate revocation of vaccination attestation. Examples include if a vaccine batch appears to be ineffective or the medical facility loses its accreditation. This information can be made available for verification of the vaccination attestation without the need to maintain the list of individuals who received the invalidated vaccination.

Global verification of VaccineGuard certificates is possible in a maximum privacy-preserving way compliant with GDPR or other strict legal frameworks:

- Once created, the vaccination certificate may be issued by the healthcare provider as a printout, sent via e-mail / mobile phone, made available for download, etc., to the patient. Only the issuer and the patient need have a copy of the certificate - no central database is required. Moreover, VaccineGuard preferably stores only the *hash* of verification data, which may be computed using any known and preferably cryptographic hash function such as one from the SHA family.

- Only the patient needs to and should be carrying and transporting the vaccination certificate, since all health and personally identifiable data are processed at her consent and no sharing is needed between the original issuer and the verifier

- A verifier (a border control officer, for example) may receive personal information like photo-ID accompanying the certificate directly from the patient at her consent. Only proof of authenticity and hashed non-personal certificate data needs to be verified against KSI blockchain and VaccineGuard.

In such a way, VaccineGuard's globally verifiable certificate solution facilitates personal control of health data, while providing assurance of the reliability of data on the certificate and that it applies to the same person possessing the connected photo-ID.

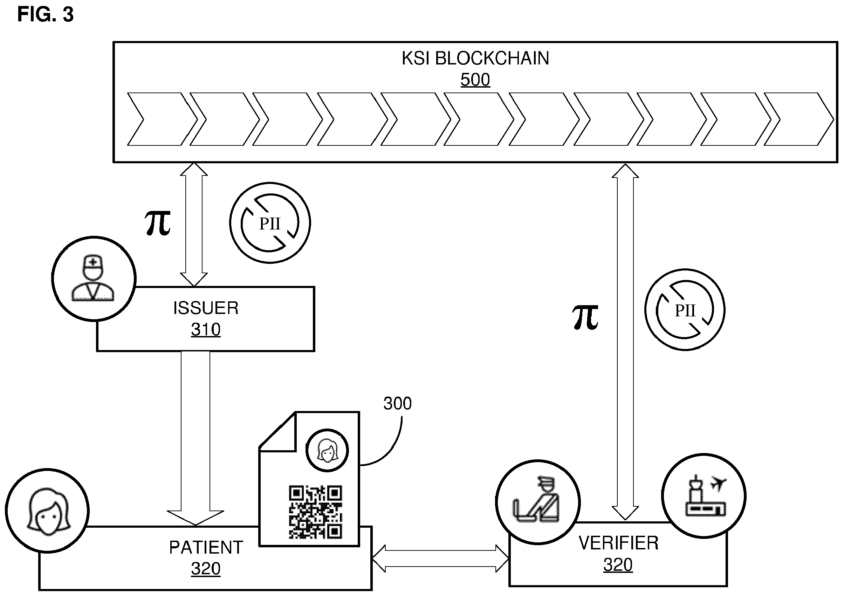

Figure 3 illustrates the procedure of provably recording information generated by the certificate Issuer 310 in the KSI blockchain 500, which will entail obtaining a KSI signature for that information, as well as the verification procedure, in which a Verifier 320 checks presented information by Patient 320 may confirm the validity of the KSI signature of the Issuer information. Figure 3 also illustrates that the KSI blockchain, that is, the KSI signature infrastructure, provides cryptographic proof π of information input into it, but does not require actual PII to do so.

Creating a vaccination record and a respective vaccination certificate by a trusted healthcare provider also enables linking a vaccine supply chain with that of a physical person in that an immutable artefact of a unique vaccine used for concrete vaccination may be anchored using its unique serial number.

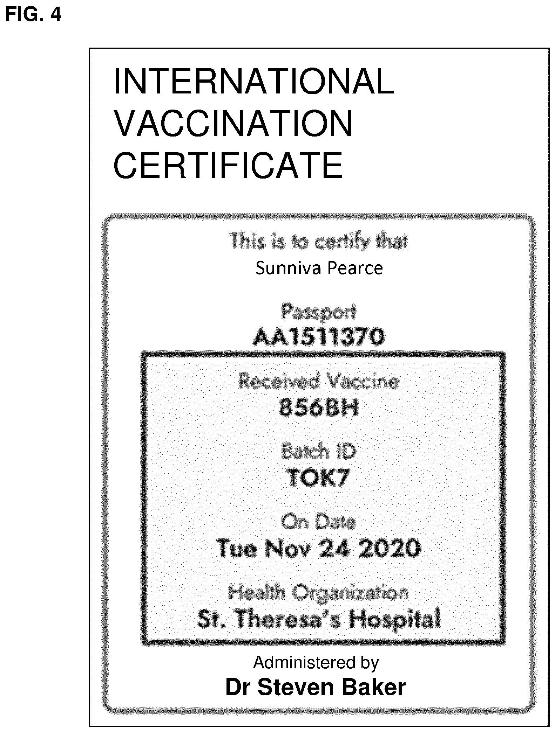

The vaccination record or vaccine certificate preferably contains information about the "last mile" of the vaccine use, such as where, when and which specific vaccine was actually administered. Figure 4 illustrates just one example of the raw patient- and "last mile" vaccine-related information that may be included on the vaccine certificate. As described and illustrated below, however, the "raw" patient information is preferably not included on the certificate, but rather a cryptographically secure but recoverable representation of it.

VaccineGuard is flexible in terms of allowing the certificate document to carry only the information that is deemed necessary and appropriate from the perspective of patient privacy. Thus, it is possible to add cryptographic proof (such as KSI signatures) to the existing, extensive data model suggested for the International Certificate of Vaccination (ICV) (often referred to as the "Yellow Card") or keep only the patient's ID and cryptographic verification proof. However, since the information about the last mile of the vaccine supply chain is cryptographically sealed for immutability, it is still possible to aggregate the counts and other relevant information about individual vaccinations, without harvesting personal information (see below), with high accuracy and in near real-time.

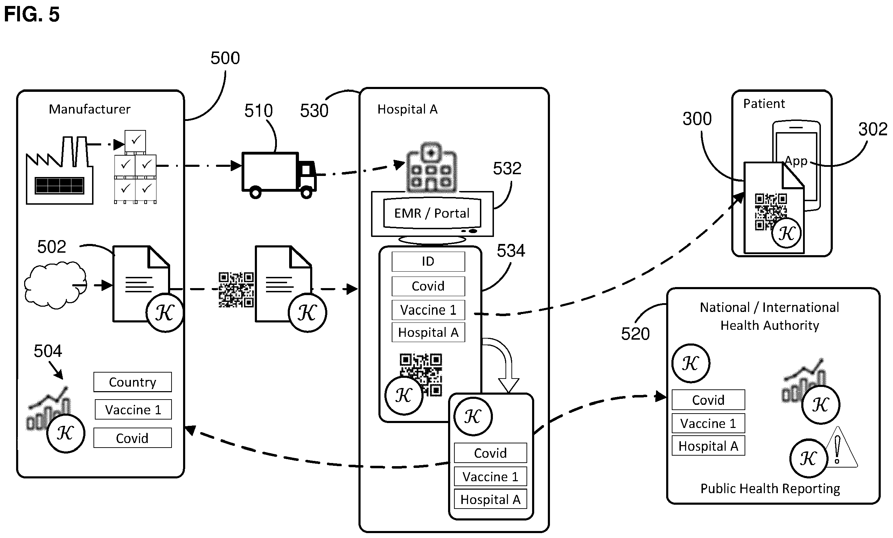

VaccineGuard also may implement a supply chain information management platform that enables end-to-end verifiable connections between all individual activities performed by various system participants, all following their autonomous but intertwined workflows. Figure 5 illustrates this possibility. In Figure 5, a manufacturer 500 produces physical vaccine (dash-dotted line shows path) and prepares it for shipment 510 to, for example, a hospital 530, which may have an electronic medical records portal 532 for entering database records 534 identifying, for example, what the vaccine is for, its identifier (batch, etc.), where it is being used (Hospital A), etc. The manufacturer will typically also receive back documentation 502 and statistical or other information 504.

VaccineGuard may be used to implement a "digital twin" (shown as dashed lines) of every individual vaccine. This "twin" may be created when, for example, a vaccine is prepared for shipment out of the manufacturing site 500, when it enters a country, for example, upon delivery to a public health authority 520 responsible for the vaccination campaign, etc.

For patients, VaccineGuard is supplied together with a mobile application 302 or web UI that enables fast capture of a unique serial number (such as the known GS1 number) from a package and verify it to "upstream" artefacts recorded by VaccineGuard about the origin and supply chain history of the given vaccine. The flexibility of the solution allows connection of just the two "end-points" (for example the manufacturer's repository and the vaccination site, without the need for any wholesaler to participate) and also enables real-time awareness.

As vaccine count and allocation may be captured by individual vaccinations, the counterfeit or diversion of vaccines is easily discoverable. The ease of revoking attestations of vaccination that are administered improperly will discourage individuals from seeking them out, and medical suppliers from providing them. Furthermore, while a vaccination attestation contains personal information, such information is not necessary for the management of supply chain integrity. Any misbehavior within the supply chain, before the vaccination procedure, can be traced, after the fact, to those end recipients affected, since the individual vaccination certificates may be revoked. Individuals who are affected in this manner will be aware, and can seek redress. This also allows anonymous monitoring of batch allocations. This mechanism provides means to control the supply chain, and ensure transparency from all sides.

In Figure 5, examples of information that may receive a cryptographic proof (such as a KSI signature) is marked with a 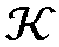 within a circle.

VaccineGuard also enables real-time reporting and aggregation information for decision makers, while still protecting patient privacy. This is provided via a secure, distributed and privacy-preserving data exchange service between VaccineGuard network participants.

### General service design and architectural criteria

In the context of embodiments that provide a vaccination certificate, it is preferable to be guided by the following general criteria and principles as suggested by the World Health Organization on Smart Vaccination certificate:
- Inclusion by design: it should be possible to read vaccination certificate information manually. The certificate holder must be able to confirm with some level of assurance that he/she has had the vaccine, without a permanent connection to the internet and/or the availability of any servers outside the jurisdiction of the validating certificate issuer.
- Complementarity by design: it should be possible to issue the vaccination certificate without the need for any additional installation of infrastructure or software by the certificate Issuer (for example, it is possible to instead use a Software-as-a-Service platform (SaaS)). However, it should also be easy to integrate into the certificate Issuer's computer systems (for example, via API calls).
- It should be possible to assume that the healthcare providers certified/authorized for the medical procedure of vaccination can also act as trusted certificate issuers, to the extent that any certificate verifier may assume that both the medical procedure and Vaccination certificate confirming it are true.
- Privacy by design: it should be possible to verify the authenticity of the vaccination certificate with a simple smartphone or computer (such as via a Web application or a mobile app) but without any need to access databases containing personal and/or medical information - or even through a trusted third-party (that is, the vaccination certificate data is presented "off-line"); any personal data processing must respect individual privacy and consent.
- Validity by design: it should be possible to validate a vaccination certificate universally (across businesses, and national and geographic boundaries) and independently from the certificate holder, issuer or verifier, using trusted electronic and governance solutions.
- Trust by design: it should be possible to secure and audit the integrity of both the vaccination certificate data and its issuance process by a technology - not by human activity - to prevent any counterfeit or fraud of either digital or paper documents.
- It should be possible to change vaccination validity information post factum: nurses can turn out to be fraudulent, batches of the vaccine can turn out to be faulty or ineffective, or the immunization period can change, for example.
- Resilience by design: it should be interoperable and adaptable with and between the existing technological solutions operated by any system, organization, country, culture or environment, without the need to build new IT infrastructure; it should be possible to govern issuance of the credentials locally without the need to rely on a globally centralized technological solution.

It is assumed that the patient will use the same identifying document(s) when presenting the proof of vaccination and when getting the vaccination; otherwise, verification of the corresponding digital signature will fail.

For the sake of properly managing privacy, data should preferably be stored appropriately. In embodiments of VaccineGuard, data may reside wherever the data owner chooses. The data may be kept locally on a commercial computing device. As just a few examples, it may be stored on a smartphone or laptop, or in a database within a hospital network; it can be hosted in a specific and acceptable cloud service provider; etc.

What follows is the description of a "traditional" model, where all personal and health data processing takes place within the existing IT infrastructure of a healthcare provider or by an authorized personal health data holder (for example, an electronic medical record vendor or national health information system). The invention is not limited to use in such a traditional model environment, however.

The VaccineGuard solution manages three types of data or data objects:

### 1) Vaccination records and vaccination certificates

These are created by authorized healthcare organizations to prove events of vaccination administration and full vaccination status.

Vaccine records, stored by health organizations for record keeping and verifying vaccination progress while vaccine certificates are given to citizens.

Vaccination certificates preferably do not contain personally identifiable information other than, optionally, the patient's name, signature, etc., but do contain a mechanism to link the vaccine certificate to a valid vaccine and an authenticated citizen.

### 2) Vaccine Data

Vaccine data provides proof of validity for the specific vaccine and specific contextual information such as, for example, how many doses the specific serial number stands for, how many vaccine doses are required to reach adequate immunity, etc. this includes possible counterfeits and the inventorying or rejection of vaccine chain of custody

### 3) Vaccination reporting data

Vaccine reporting data is anonymous information about a vaccination event that does not contain any reference to a physical person but rather only a cryptographic link to a unique event that prevents double counting. This may then be used in aggregated vaccination reports.

Examples of those who may wish to process data include, healthcare organizations, public health authorities, citizens/patients, and verifiers.

Healthcare organizations that are authorized (see above) to have a VaccineGuard account may use the software to certify vaccination records and generate vaccination certificates. The data is created on the machine, which is controlled and managed by the organization (for example within a single physical network), and may also be stored within the healthcare organization's digital infrastructure. At least from the perspective of VaccineGuard, there is no need for the data of the vaccine certificate ever to be stored centrally or in some other central data structure. The certificate may, however, be sent directly to the patient by the healthcare organization or exchanged with national authorized entities, if relevant.

Public health authorities may receive via VaccineGuard the vaccination reporting data according to the predefined rules. Aggregation may be facilitated at different levels from a hospital to regional or national authorities to supranational entities. While the reporting data is based on a unique count of a vaccination event, preferably it does not contain any personally identifiable data and is also used in an aggregated form.

Citizens/patients can have sent by the healthcare organization a copy of their vaccination certificate, which is linked to their photo-ID or personal ID-number that was used for authentication by the healthcare organization. The vaccine certificate does not need to contain any Personal Identifiable Information (PII), but rather only a cryptographic guarantee, for its integrity (data has not been tampered) and validity (data belongs to a specific individual). The KSI system is summarized below as a good way to provide this cryptographic guarantee. This allows for confident and confidential exchange of the vaccine certificates to enable on-demand verification.

VaccineGuard protects data privacy by following industry standards for protecting PII. Vaccine certificates and records require the ability to be uniquely tied to an individual. To provide the utmost data privacy while still achieving this goal, VaccineGuard may use hashes preferably associated with the patient's unique, government-issued, identity credential, as well as "salt" if and as needed to increase entropy. This can be in the form of a government issued passport number, national ID card, personal ID-number, etc. An illustration showing the relationship is below.

The technique of "salting" is well known in the area of cryptography, and is used to increase the entropy of information. Most passport numbers, for example, consist of seven to nine alphanumeric characters, usually mostly numbers, and they are generally not generated as random numbers. Even if a passport number is hashed using a cryptographic hash function such as SHA-256, the universe of possible passport numbers is small enough that even their hash is susceptible to a "rainbow table" attack. By adding a large enough salt value to the input to the hash function, along with the passport number, the entropy of the output will be so great that such an attack will not be feasible. Nonetheless, "salting" may not be necessary in implementations in which the entropy of the hash input value is considered high enough without it. For example, the patient's associated identifying number may be long enough as is, or other information such as the patient's name may be hashed together with it, to provide sufficient entropy.

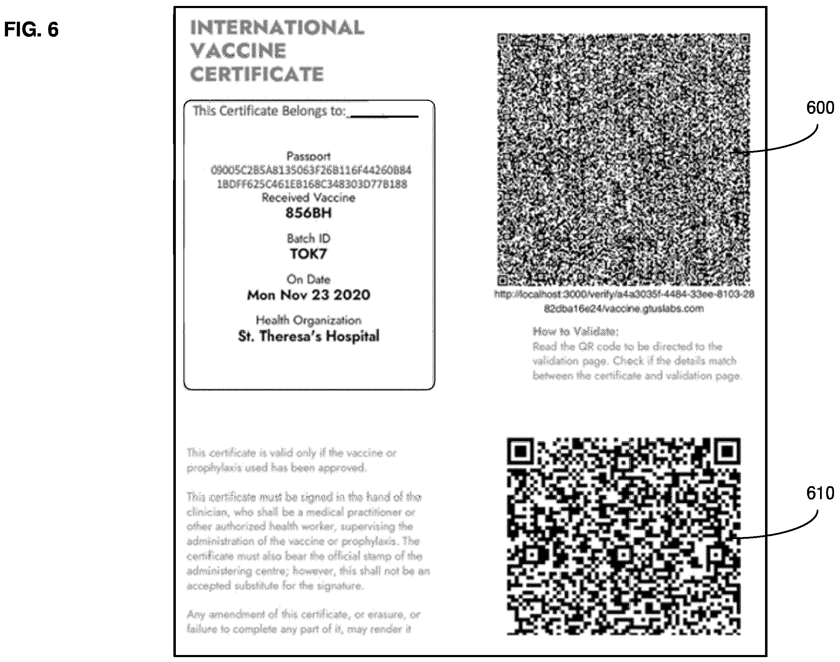

Figure 6 illustrates an example of a Vaccine Certificate that incorporates the features of embodiments of the invention. The certificate could be in any chosen form, such as a single page, a "booklet", a file displayable on a smart phone, or even reduced to be an easily portable card as long as the information printed on it is legible by both human and machine.

Compare this example with Figure 4, and note that, instead of name of the patient ("Sunniva Pearce") who received vaccine 856BH, Batch ID TOK7 on 23 November 2020 at "St. Theresa's Hospital", the patient is here identified by a cryptologic function of at least the number of her ID, such as of her ID document number, along with the salt value. In a preferred embodiment, the cryptologic function is a hash. Here, the ID document is indicated as being her passport, although any other approved ID document could be used. Note that it would also be possible to include other identifying information in the hash function as well, such as the patient's name as shown on the ID document; this is a design choice, but because cryptographic hash functions are in general non-commutative with respect to their input parameters, the convention that is used should be known and understood by verifiers as well, and the holder of the ID document and vaccine certificate must be able to provide all hashed information to a verifier.

As shown in the upper part of the left side of the illustrated certificate, ("This Certificate Belongs to: _____"), a physical signature may also be required, for example, of the medical practitioner or other authorized health worker who carried out or supervised the administration of the approved vaccine. As one alternative, the physical signature might be replaced with or augmented by a different identifier, such as an encoding of a public key of the medical practitioner.

Figure 6 also illustrates two markings. In this case, the markings are well-known QR codes, which are shown by way of example. One of the QR codes, typically one of a higher QR code version to be able to encode more information, encodes a digital signature (see below) of the hash value of the patient's identifier; the other QR code encodes the "last mile" information of the certificate, such as that illustrated in Figure 4.

Although not illustrated in Figure 6, a third marking (such as a QR code) may also be include on the certificate and include the "salt" value used in generating the hash value of the patient identifier.

### Verification

Eventually the citizen/patient will want to prove vaccination to a verifying entity. To do this, the markings (shown as the QR codes) on the vaccination certificate are required, but *not* any unencrypted/unhashed "raw" (cleartext) personal information, or any querying of a centralized database. In particular, there is no need to access the certificate issuer's original records at the healthcare organization, where the original data in the certificate might be held.

Once the citizen presents the certificate to the verifying entity, the verifying entity, using, for example, a smart phone, tablet, or other known device, scans the QRCode(s) associated with the vaccine certificate and extracts the encoded information, thereby reassembling the "digital twin" of the vaccine associated with the vaccine certificate. This will provide the data, the digital signature, and the "salt" value if included.

The patient/citizen also presents to the verifying entity the physical document that was the basis of the patient's hashed ID. Assume by way of example that this document is the patient's government-issued passport. The verifying entity then compares the physical passport number with the number encoded on the certificate. All must match in order for the vaccine certificate verification to be successful.

One way to accomplish the comparison is for an application loaded and running in the verifying entity's scanning device (such as a smart phone) to input the physical passport number, along with any other optically readable or manually entered information used in the original hash computation, extract the salt value from the QR code (if used), recompute the hash value using the same hash function as when the QR code was generated, and compare the result with the hash value encoded on the certificate.

The verifying entity may then validate the cryptographic evidence locally on the verifying entity device. In a preferred embodiment, in which information is digitally signed using a KSI signature (see below), the verifying entity will validate the authenticity of the certificate using the KSI Signature. This process does not require data exchange with IT systems or key exchanges across organizations. The verification of a KSI signature typically takes milliseconds, and can be done within the verifying entity's device, independently of any server-assisted responses. The KSI Signature is then validated using the KSI blockchain, where verifying entities can confirm the respective calendar and/or publication value, thereby proving or disproving authenticity of the signature and in turn the vaccine certificate. Note that this does not require exchange of data, but instead only cryptographic blocks, or hashes.

### KSI Signatures

Digital signatures are used in the invention. Many known signature schemes may be used as long as they can be encoded in the marking (such as QR code) and the verifier's device is configured to read it. A particularly advantageous form of digital signature, however, and the one mentioned above by way of illustration, is the one generated using the data signature infrastructure developed and marketed under the name KSI^{®} by Guardtime AS of Tallinn, Estonia. This system is described in general in U.S. Patent No. 8,719,576 (also Buldas, et al., "Document verification with distributed calendar infrastructure"). In summary, in its basic form, for each of a sequence of calendar periods (typically related one-to-one with physical time units, such as one second), the Guardtime infrastructure takes digital input records (such as data values or sets, or "messages") as inputs. These are then cryptographically hashed together in an iterative, preferably (but not necessarily) binary hash tree, ultimately yielding an uppermost hash value (a "calendar value") that encodes information from all the inputs (the tree's "leaves"). This uppermost hash value is then entered into a "calendar", which is structured as a form of blockchain ("KSI Blockchain") in that each new calendar entry is cryptographically linked with at least the previous calendar value.

For additional security, the Guardtime signatures can be extended after a number of calendar periods up through a progressively growing Merkle tree of calendar values, or a hash-chaining linkage of calendar values, to a publication value that is published in any widely witnessed manner, such as in a printed publication, an online database, in a ledger, in a blockchain, etc. It is also possible to forego the accumulation of calendar values via a Merkle tree and instead enter each calendar value into some widely witnessed data structure such as a blockchain-backed ledger; indeed, the Guardtime KSI calendar itself has a blockchain structure, and may itself be sufficient even without additional hashing using a Merkle tree and publication.

The KSI system then returns a signature (referred to here as a "KSI signature") in the form of a vector, including, among other data, the values of sibling nodes in the hash tree that enable recomputation of the respective calendar value, or extended to the corresponding publication value, if a purported copy of the corresponding original input record is in fact identical to the original input record.

As long as it is formatted according to specification, almost any set of data, including concatenations or other combinations of multiple input parameters, may be submitted as the digital input records, which do not even have to comprise the same parameters. One advantage of the KSI system is that each calendar block, and thus each signature generated in the respective calendar time period, has an irrefutable relationship to the time the block was created. In other words, a KSI signature also acts as an irrefutable timestamp, since the signature itself encodes time to within the precision of the calendar period.

One other advantage of using a Guardtime infrastructure is that there is no need to store and maintain public/private (such as PKI) key pairs - the Guardtime system may be configured to be totally keyless except possibly for the purposes of identifying users or as temporary measures in some implementations in which calendar values are themselves combined in a Merkle tree structure for irrefutable publication. Another advantage is less apparent: Given the signature vector for a current, user-presented data record and knowledge of the hash function used in the hash tree, an entity will be able to verify (through hash computations as indicated by the signature vector) that a "candidate" record is correct even without having to access the signature/timestamping system at all: If the same information is made available as formed the input to receive the KSI signature, then it is certain that, using the presented, purportedly correct, information to recompute "upward" through the KSI hash tree by iteratively hashing with the values in the signature, if the same root value is obtained (which is also included in the KSI signature), then the presented information is in fact identical to the input originally used to generate the signature.

Yet another advantage of the Guardtime infrastructure is that the digital input records that are submitted to the infrastructure for signature/timestamping do not need to be the "raw" data; rather, the raw data may optionally be combined with other input information (for example, the salt value, or such information as input server ID, Patient ID, location, etc.) and then hashed. Given the nature of cryptographic hash functions, what gets input into the KSI system, and thus ultimately into the calendar blockchain, cannot be reconstructed from the hash, or from what is entered into the calendar blockchain. If an entity, in compliance with GDPR, for example, assures a user that his raw data has been "forgotten" in its database (an action that itself may be recorded in the blockchain), then the information encoded in the Guardtime KSI blockchain structure may remain as is, since it is impossible to deduce what raw data led to it. This is in contrast to many existing blockchain solutions, whose blocks include raw data: Deleting or altering any block in such a blockchain renders the chained linking data invalid; alternatively, creating a forked, that is, parallel blockchain path to accommodate a deletion weakens or destroys the security and trustworthiness of the system as a whole.

### Alternative markings

The markings on the certificate encode information in a machine-readable (including via software, such as by processing a captured image). The markings mentioned primarily in this disclosure are in the form of Quick Response (QR) Codes; these are advantageous because they have already gained nearly worldwide acceptance for encoding information in an optically readable form. Moreover, QR code-reading software is incorporated into many devices such as "smart" telephones and tablet computers and automatically extracting and optionally displaying and acting on the encoded information.

Different versions of the QR code (as well as of similar codes) can encode different numbers of alphanumeric characters, depending on the level of error correction included and the granularity of the code pattern itself. System designers will know which version of a given code to use for the different data sets encoded on a certificate based on such factors as how much information it is to encode and under what conditions and using what devices users will need to read the code. For example, if the marking is to encode only the hash of the "last-mile" information on the vaccination certificate, then a lower version of the QR or similar code might be appropriate, whereas a higher version QR code may be necessary to encode a full KSI signature.

Although QR codes have gained wide acceptance, the invention does not require any particular encoding scheme for the markings and in fact there are other schemes in use even today. One such alternative is Microsoft Tag. Some other alternatives include Data Matrix and PDF417. Still other alternatives are non-barcode-based schemes that might, for example, include markings made up of alphanumeric characters in a particular pattern or shape that can be captured from images and interpreted using OCR to extract the encoded document identifier. The document identifier could then optionally be encoded into the marking in the form of a cryptogram that only dedicated reading software can decode, thereby providing an additional level of security and making it harder for malicious actors to falsify the marking.

It is also not necessary for the markings to be visual/optical, although this will in general be the easiest to implement. One alternative would be to "mark" the certificate electrically or digitally, such as encoding its identifier in a read/write RFID tag or a programmable smart chip, such as is found in biometric passports, many bank cards, etc.

### Other types of certificates

The invention is described above for use in providing a verifiable vaccination certificate. The techniques described for that use case may, however, also be applied in any use case in which one wishes to verify that some document presented by a holder is indeed associated with the holder. Similarly, the invention is not limited to use in the context of vaccines; rather, any type of "event" may be verifiably certified with the techniques described above. Here, "event" is to be interpreted broadly to include anything that can be associated with the action of any entity or thing that can be assigned a digital identity (such as a serial number or other identifier), such as the manufacture, production, or use of something, such that the thing or action needs to be verifiably tracked, especially to the point of delivery to a user.

## Claims

1. A method for creating and verifying a certificate attesting to an event, comprising:
inputting event-defining information (210, 220, 230) including an identifier of an end user;
obtaining for the event-defining information at least one digital signature;
generating at least one optically readable marking (600, 610) that encodes the at least one digital signature;
applying the marking to a medium that bears the certificate;
in which the digital signature is obtained by submitting the event-defining information as an input to a hash tree infrastructure (500), in which the digital signature comprises a vector of sibling values enabling recomputation upward through the hash tree infrastructure to a root value that represents an uppermost value of the hash tree infrastructure having a plurality of tree input values submitted during an accumulation period at a corresponding calendar time, said root value being included in the digital signature;
whereby a later purportedly authentic representation of the event-defining information may be verified as being valid if, upon recomputing a hash tree path represented by the sibling values, from the purportedly authentic representation of the event-defining information through the hash tree, the same root value is obtained as is contained in the digital signature;
whereby the correctness of the event-defining information encoded in the marking in the certificate is verifiable without knowledge of or communication with an external database of unencoded personal identifiable information of the end user.

2. The method of claim 1, in which the event-defining information relates to administration of a vaccine to the end user (320) and includes an encoding of an identifier of the end user and identifying information of the administered vaccine.

3. The method of claim 2, further comprising including a salt value along with the identifier of the end user upon obtaining the digital signature.

4. The method of any of claims 2-3, further comprising
generating a first optical marking encoding a first digital signature of the identifier of the end user and a separate, second optical marking encoding information identifying the vaccine and administration-related data;
applying both the first and second optical markings to the certificate.

5. The method of claim 4, in which the administration-related data includes data identifying the vaccine administered, the vaccine administration protocol, and/or the authorized healthcare provider that administered the vaccine.

6. The method of claim 3, further comprising generating a third optical marking encoding the salt value and including the third optical marking in the certificate.
